Europäisches Patentamt

European Patent Office

Office européen des brevets

(11) Publication number: **0 225 581**
A2

## (12) EUROPEAN PATENT APPLICATION

(21) Application number: **86116691.6**

(22) Date of filing: **01.12.86**

(51) Int. Cl.⁴: **A 61 K 47/00, A 61 K 39/395**

(30) Priority: **30.11.85 JP 270195/85**
**17.01.86 JP 8703/86**

(43) Date of publication of application: **16.06.87**
**Bulletin 87/25**

(84) Designated Contracting States: **BE CH DE ES FR GB LI NL SE**

(71) Applicant: **GREEN CROSS CORPORATION, 15-1, Imabashi 1-chome Higashi-ku Osaka-shi, Osaka (JP)**

(72) Inventor: **Hirao, Yutaka, 14-1-606, Terauchi 2-chome, Toyonaka-shi Osaka (JP)**
Inventor: **Uriyu, Katsuhiro, 601-32, Daifukunakatsumichi 3-chome, Sakurai-shi Nara (JP)**
Inventor: **Uemura, Yahiro, Maison Hirakata 215 5-18, Mitsuya-cho, Hirakata-shi Osaka (JP)**
Inventor: **Takechi, Kazuo, 25-308, Kawanakashin-machi, Daito-shi Osaka (JP)**

(74) Representative: **Hansen, Bernd, Dr.rer.nat. et al, Hoffmann, Eitle & Partner Patentanwälte Arabellastrasse 4, D-8000 München 81 (DE)**

(54) Method for the heat-treatment of immunoglobulins and immunoglobulin product.

(57) The present invention provides a method for the heat-treatment of virus-containing immunoglobulins in a dry state and in the presence of at least one stabilizer selected from sugar alcohols and disaccharides for the inactivation of the virus contained therein. By the present dry heat-treatment, the virus contained in the immunoglobulin can fully be inactivated and further, the stability and the water-solublity of the heat-treated immunoglobulin can be improved.

## METHOD FOR THE HEAT-TREATMENT OF IMMUNOGLOBULINS AND IMMUNOGLOBULIN PRODUCT

### FIELD OF THE INVENTION

The present invention relates to a method for the heat-treatment of immunoglobulins for the inactivation of viruses contained therein and to an immunoglobulin product obtainable hereby.

### BACKGROUND OF THE INVENTION

One satisfactory method for the inactivation of viruses which contaminate an albumin or the like plasma proteins is a method for heat-treating an aqueous solution of the virus-containing proteins (which is referred to as a liquid-heating method, hereunder), as based upon Murray, et al, The New York Academy of Medicine, 31(5), 341-358 (1955). This liquid-heating method has been widely utilized over a long period of time up to the present, and the virus-inactivation effect of the liquid-heating method has been proven from an epidemiological aspect.

However, the class of plasma proteins like albumin, which maintain their activity when heated in a liquid state, is extremely limited and in particular, some plasma proteins which have a physiological activity or a biological activity are extremely sensitive to heat and easily deteriorate when heated, resulting in reduction of their activity or in complete deactivation thereof.

On the other hand, apart from the liquid-heating method, it has been proven in an experiment by the use of a model of a blood coagulation VIII-factor, that when the plasma proteins are heat-treated in a dry state, free from water or containing very little water, (such a heat-treatment being referred to as a dry heat-treatment hereunder), the decrement of protein activity is remarkably inhibited, as compared with the liquid-heating method (refer to U.S. Patent 4,556,558 : Rubinstein).

In general, however, the decrement of the activity of the plasma proteins is inevitable even when subjected to the dry heat-treatment, if no stabilizer is added thereto, and as a matter of fact, the solubility of the plasma proteins in water and the state of the stability and clarity of the solution thereof as dissolved therein often deteriorate.

Regarding the functional mechanism of the inactivation of viruses by heating, it has been said that the inactivation is mainly based upon modification of the protein component of the virus by the liquid-heating method, while the inactivation in the dry heat-treatment is believed based upon oxidation of the lipid component of the virus; thus, the viruses are injured to an extent that they lose their pathogenic activity. It is, therefore, suggested that the virus inactivation modes of the two heating means are basically different from each other, although the virus-inactivating mechanisms apparently do partly overlap to each other. (Refer to Rahn, Physical Methods of Sterilization of Macroorganisms, Bact. Rev., 9, 1-47, (1945).)

One object of the present invention is to provide a method for the heat-treatment of immunoglobulins to inactivate viruses contained therein without inactivating the immunoglobulins themselves.

Another object of the present invention is to provide a method for the heat-treatment of immunoglobulins to inactivate viruses contained therein without adversely affecting solubility of the immunoglobulins in water nor the continued clarity of the solution thereof in water.

The present inventors have found an improvement in the dry heat-treatment of immunoglobulins to inactivate the viruses contained therein without causing loss of the activity of the immunoglobulins. In particular, the present inventors have found that dry heat-treatment of the immunoglobulins can be carried out in the presence of certain stabilizer compounds to remarkably stabilize the immunoglobulins and, in addition, that the immunoglobulins thus treated by the dry heat-treatment are highly soluble in water and form a water solution of good stability and clarity.

## SUMMARY OF THE INVENTION

The present invention provides a method for the heat-treatment of immunoglobulins in which a virus-containing immunoglobulin is heated in a dry state in the presence of at least one stabilizer compound selected from sugar alcohols and disaccharides, until the virus contained therein is inactivated. By the present dry heat-treatment method, the viruses contained in the immunoglobulins are inactivated and further, the stability and the water-solubilty of the immunogobulins are improved over the known dry heat-treatment process.

## DETAILED DESCRIPTION OF THE INVENTION

The immunoglobulins to be heat-treated in accordance with the method of the present invention are those having biological activity and physiological activity as immunoglobulins and, for example, those obtained by the fractionation of plasma proteins.

The immunoglobulins are exemplified to include those derived from human, horse and mouse, and these may be either a polyclonal antibody or a monoclonal antibody and are, preferably, IgG, IgA or IgM types.

The present invention is practiced, in general, by first freeze-drying the immunoglobulin solution and then heating the solution under the condition of a moisture content of 0.05 to 3%, during which the presence of a specifically determined stabilizer is effective for the intensification of the stability of the heat-treated immunoglobulin and for the improvement of the solubility thereof, as well as of the state of stabililty and clarity of the resulting immunoglobulin solution.

The stabilizer which can be used in the method of the present invention is at least one sugar alcohol or disaccharide. Preferred examples of the sugar alcohols include sorbitol, mannitol, xylitol, galactitol and the like; and those of the disaccharides include saccharose, maltose, lactose and the like. These can be used in combination.

The amount of the stablizer to be used is a concentration effective as a stabilizer, with the following set forth as guidelines, for example: The concentration of the stabilizer is regulated to be about 1 to 5 w/v%, more preferably about 2 to 3 w/v% or so, per about a 0.01 to 2 w/v%-solution of a monoclonal immunoglobulin or per about a 2 to 8 w/v%-solution of a polyclonal immunoglobulin. More specifically, the stabilizer is used in an amount of 1 to 5 weight parts, more preferably 2 to 3 weight parts, per 0.01 to 2 weight parts of a monoclonal immunoglobulin or per 2 to 8 weight parts of a polyclonal immunoglobulin. The addition of the stabilizer within the above concentration range is most preferred to attain the best balance between the stabilization effect, the water-solubility, the state of the solution and the formation of the preparation.

The immunoglobulin is generally used in the form of a freeze-dried product, and the stabilizer is preferably added prior to the freeze-drying treatment of the plasma proteins.

The stabilizer may be removed after the dry heat-treatment of the present invention, but is preferably left as such in the plasma protein-containing preparation without being removed therefrom.

Thus, in accordance with this invention, the stability of the immunoglobulin during the freeze-drying thereof and the preservation stability of the preparation thereafter are improved.

The heating temperature in the heating treatment is generally about 30°C to 100°C, preferably about 60°C or so; and the heating time is generally about 10 minutes to 200 hours, preferably 10 to 100 hours or so, as is known in the art.

The viruses to be inactivated in accordance with the heat-treatment of the present invention are those which are known to contaminate human plasma proteins, and in particular, hepatitis viruses or the like.

The heat-treatment of the present invention is preferably carried out under the condition of an inert gas atmosphere, whereby the stability obtained in the heating may further be elevated. Examples of the inert gases which can be used include nitrogen gas, argon, helium and the like.

The purity of the immunoglobulins and the heat- resistance thereof are not believed to be directly related and therefore, the stabilization effect obtained by the stabilizer use does not vary depending upon the degree of purification of the immunoglobulins. Accordingly, the heat-treatment of the present invention may be carried out at or after any stage of the purification of the immunoglobulin.

The "dry state" in the dry heat-treatment of the present invention means a substantially water-free state and is preferably a state containing the least amount of water as possible. The content of water of the "dry state" is generally 3% or less, preferably 1% or less, and is suitably 0.05 to 3% or so, based on the weight of the immunoglobulin.

According to the present invention, it is possible to inactivate the viruses which contaminate blood preparations without a significant loss of the activity of the plasma proteins, which are valuable blood preparations, and therefore, the method of the present invention is an industrially useful and advantageous method for the manufacture of plasma protein-containing preparations.

The present invention will be explained in greater detail by reference to the following examples and experiments, which, however, are not intended to be interpreted as limiting the scope of the present invention.

EXAMPLE 1

Fr-II (IgG fraction) was obtained from a normal human plasma in accordance with Cohn's cold alcohol fractionation method. One (1) kg of the Fr-II paste was dissolved in 1.5 liters of a cold water to make a 5 w/v% of IgG solution, and 30 g of sorbitol was added to the resulting solution so that the concentration of sorbitol was 2 w/v%. The pH of the IgG solution was adjusted to be 6.3 to 6.5 and then the solution was freeze-dried. The water-content in the freeze-dried product was 0.8%. The thus freeze-dried IgG powder was heat-treated at 60°C for 72 hours. The heat-treated powder was tested on the items of solubility, HBsAg-antibody value, measles antibody value, diphtheria antitoxin value, cellulose acetate membrane electrophoresis and gel filtration, while compared with an IgG powder which was not heat-treated (control). As a result, no significant change was noticed in the heat-treated powder as compared to the control, which means that the human IgG was stable when heat-treated in the presence of a stabilizer as disclosed herein.

EXAMPLE 2

From a Fr-III fraction as obtained by Cohn's cold alcohol fractionation method, there was obtained pure human IgA by salting out, acrinol fractionation.

Twenty (20) g of saccharose was added to 1ℓ of the 5 w/v%-solution of the human IgA so that the concentration of saccharose was 2 w/v% and then it was freeze-dried. The water-content in the freeze-dried powder was 2% or less. This powder was heated at 60°C for 72 hours and tested on the items of solubility, IgA concentration, measles antibody value and cellulose acetate membrane electrophoresis. The results of these tests on the present heat-treated powder were compared with those on the powder which was not heat-treated (control), and as a result, it was confirmed that the powder processed in accordance with the present invention was stable even after being heat-treated.

EXPERIMENT 1 (Kind of Stabilizer):

Fr-II (IgG fraction) was obtained from a normal human plasma in accordance with Cohn's cold alcohol fractionation method. Twenty (20) g of each stabilizer as shown in Table 1 below was added to 1ℓ of a 5 w/v%-solution of the IgG fraction so as to make the concentration of each stabilizer being 2 w/v% and, after the pH of the resulting solution was adjusted to 6.3 to 6.5, this was freeze-dried. The IgG powder was heat-treated at 65°C for 96 hours, and then tested on the items of solubility, diphtheria antitoxin value, measles antibody value and anticomplement value. The tests were carried out in accordance with Standards of Biological

Preparations except for the solubility test.

More particularly, the tests were carried out as follows.

Solubility

Solubility was observed with the cyc.

Diphtheria Antitoxin Value

The value was determined by a method of intradermal injection into rablit (in vivo).

Standard diphtheria antitoxin (its potencies known) and each sample to be tested were gradually diluted and a predetermined amount of diphtheria toxin was added thereto. Each resulting mixture was intradermally injected into a rabbit and after 48 hours flush area is measured. The diameter thereof having 10 mm or more was defined as positive. Each maximal dilution of standard and samples defined as positive was determined. The potencies of samples were determined by comparing the value of standard with those of samples.

Measles Antibody Value

This value was determined by hemagglutination test (in vitro).

Standard serum containing measles antibody and each sample to be tested were gradually diluted and a constant amount of measles antigen and hematocyte of green monkey were added thereto. Each mixture was incubated so as to conduct hemagglutination. Each maximal dilution of standard and samples defined as positive in hemagglutination test was determined.

The pontencies of samples were determined by comparing the values of samples with the value of standard.

Anticomplement Value

This value was determined by anticomplement negative test. 1 ml of a solution containing 100 units of guinea pig complement and 3 ml of an appropriate buffer were added to 1 ml of standard complement and each sample. The remaining amount of complement contained in standard and the sample after heating at 37°C for 1 hour was referred to $\underline{a}$ and $\underline{b}$, respectively. The amount of inactivated complement (anticomplement value) was determined by the following formula

Anticomplement Value = $\underline{b}$ – $\underline{a}$

The results are given in Table 1.

Table 1

| Stabilizer | Solubility | Diphtheria Antitoxin Value (IU/100mg) | Measles Antibody Value (IU/100mg) | Anti-complement Value (CH$_{50}$/ml) |
|---|---|---|---|---|
| FR-II (standard), before heat-treatment | - | 3-4 | 27 | 16 |
| No addition (control) | C | 2-3 | 20 | 46 |
| Single stabilizer | | | | |
| Saccharose | A | 3-4 | 28 | 20 |
| Sorbitol | A | 3-4 | 26 | 26 |
| Maltose | A | 3-4 | 25 | 18 |
| Mannitol | B | 3-4 | 26 | 23 |

\* A : No isoluble precipitates existed.

  B : A little insoluble precipitates existed.

  C : Insoluble precipitates existed.

0 225 581

The above results show that the dry heat-treatment in the presence of the stabilizer is more effective for improvement of stability than that in the absence of the stabilizer (control).

EXPERIMENT 2 (Amount of Stabilizer):

Each stabilizer was added to 1 ℓ of a 5 w/v%-aqueous solution of the Fr-II (IgG fraction) as prepared in accordance with Example 1, in an amount as shown in Table 2 below, and after the pH value of the resulting solution was adjusted to 6.3 to 6.5, this was freeze-dried. Afterwards, the powder was tested in the same manner as in Experiment 1. The results obtained are given in Table 2.

Table 2

| Stabilizer | Added Amount (g) | Solubility | Diphtheria Antitoxin Value (IU/100mg) | Measles Antibody Value (IU/100mg) | Anti-Complement Value (CH$_{50}$/mo) |
|---|---|---|---|---|---|
| FR-II (standard) | – | – | 3-4 | 27 | 16 |
| No addition (control) | 0 | C | 2-3 | 20 | 46 |
| Single stabilizer | | | | | |
| Saccharose | 5 (0.5 w/v%) | A | 2-3 | 23 | 25 |
| " | 10 (1.0 " ) | A | 3-4 | 23 | 21 |
| " | 20 (2.0 " ) | A | 3-4 | 26 | 20 |
| " | 30 (3.0 " ) | A | 3-4 | 26 | 21 |
| " | 40 (4.0 " ) | A | 3-4 | 27 | 20 |
| " | 50 (5.0 " ) | A | 3-4 | 27 | 20 |
| Mannitol | 10 (1.0 " ) | A | 2-3 | 23 | 25 |
| " | 20 (2.0 " ) | A | 3-4 | 27 | 23 |
| " | 30 (3.0 " ) | A | 3-4 | 27 | 24 |
| " | 40 (4.0 " ) | A | 3-4 | 27 | 25 |
| " | 50 (5.0 " ) | C | 2-3 | 20 | 25 |

A: No insoluble precipitates existed.
B: A little insoluble precipitates existed.
C: Insoluble precipitates existed.

It is confirmed from the results that the effective amount of the stabilizer to be used in the dry heat-treatment of the present invention is suitably within the range of 1 to 5 w/v%.

EXPERIMENT 3

Twenty (20) g of saccharose was added to 1ℓ of a 5 w/v%-aqueous solution of Fr-II (IgG fraction) as prepared in accordance with Example 1 so as to make the concentration of saccharose being 2 w/v%, and a virus-containing suspension in 0.05M-phosphate buffer (pH 7.1) was added thereto, uniformly blended therewith and then freeze-dried.

After completion of freeze-drying, the internal pressure was made normal with nitrogen gas and the content was sealed and immersed in a hot bath of 60°C and heated. In the heat-treatment, 10 to 15 minutes were required until the inner temperature rose to 60°C, and in practice, the heating time was prolonged by 30 minutes in each case.

The virus infection was measured by means of a plaque-forming method, as described in L. B. Epstein, "Assay of Human Immune Interferon from Lymphocyte Macrophage Cultures by a Virus Plaque Reduction Method", in Manual of Clinical Immunology, pp120-128, American Society of Microbiology, 1976.

The results are given in Table 3.

Table 3

| Virus | Before Freeze-Drized | Virus Infection *1 | | | | |
|---|---|---|---|---|---|---|
| | | Heating Time After Freeze-Dried | | | | |
| | | 0 hr | 10 hrs | 24 hrs | 48 hrs | 72 hrs |
| Vesicular stomatitis virus | $1.0 \times 10^6$ | $2.3 \times 10^5$ | $<10$ *2 | $<10$ | $<10$ | $<10$ |
| Chikungunya virus | $1.7 \times 10^6$ | $7.5 \times 10^5$ | $<10$ | $<10$ | $<10$ | $<10$ |
| Mumps virus | $2.2 \times 10^6$ | $4.5 \times 10^5$ | $<10$ | $<10$ | $<10$ | $<10$ |
| Herpes simplex virus | $1 \times 10^6$ | $5.8 \times 10^3$ | $<50$ *3 | $<50$ | $<50$ | $<50$ |
| Sindbis virus | $3.2 \times 10^7$ | $1.3 \times 10^7$ | $5.5 \times 10^3$ | $<50$ | $<50$ | $<50$ |

Notes:

   *1    :    Plaque forming (Unit/ml)

   *2, *3,:    10,   50;   Under detectable limit

0 225 581

As shown in the result of Table 3, each virus plaque was measured under detectable limit and it was found that the infectivity of each virus was lost by heating at 60°C for 24 hours or more.

While the invention has been described in detail and with reference to specific embodiment thereof, it will be apparent to one skilled in the art that various changes and modifications can be made therein without departing from the spirit and scope thereof.

## CLAIMS

1.     A method for the heat-treatment of virus-containing immunoglobulins, comprising heating the virus-containing immunoglobulin in a dry state in the presence of at least one stabilizer selected from the group consisting of sugar alcohols and disaccharides at a temperature and time sufficient to inactivate said virus.

2.     The method for the heat-treatment of virus-containing immunoglobulins as claimed in claim 1, wherein the virus-containing immunoglobulin is heated under the condition of a moisture content of 3% or less.

3.     The method for the heat-treatment of virus-containing immunoglobulins as claimed in claim 1, wherein the immunoglobulin to be treated in one derived from human, horse or mouse.

4.     The method for the heat-treatment of virus-containing immunoglobulins as claimed in claim 3, wherein the immunoglobulin derived from human or mouse is a polyclonal antibody or monoclonal antibody.

5.     The method for the heat-treatment of virus-containing immunoglobulins as claimed in claim 1, wherein the immunoglobulin to be treated is IgG, IgA or IgM.

6.     The method for the heat-treatment of virus-containing immunoglobulins as claimed in claim 1, wherein the concentration of stabilizer to be added to the immunoglobulin is regulated to be about 1 to 5 w/v% per about a 0.01 to 2 w/v%-solution of a monoclonal immunoglobulin or per about a 2 to 8 w/v%-solution of a polyclonal immunoglobulin.

7.    The method for the heat-treatment of virus-containing immunoglobulins as claimed in Claim 1, wherein the stabilizer is used in an amount of 1 to 5 weight parts per 0.01 to 2 weight parts of a monoclonal immunoglobulin or per 2 to 8 weight parts of a polyclonal immunogloblin.

8.    An immunoglobulin product obtainable by the process of claim 1, said product containing said at least one stabilizer.

9.    A stable solution of the immunoglobulin product of claim 7.

10.    The method for the heat-treatment of virus-cotaining immunoglobulins as claimed in claim 1, wherein said stabilizer is a sugar alcohol selected from the group consisting of sorbitol, mannitol, xylitol and galactitol.

11.    The method for the heat-treatment of virus-containing immunoglobulins as claimed in claim 1, wherein said stabilizer is a disaccharide selected from the group consisting of saccharose, maltose and lactose.

12.    The method for the heat-treatment of virus-containing immunoglobulins as claimed in claim 1, wherein said virus-containing immunoglobulin has been freeze dried together with said stablizer prior to said heating.